Europäisches Patentamt

**European Patent Office**

**Office européen des brevets**

(19)

(11) Publication number:

**0 156 299**

**A2**

(12)

# EUROPEAN PATENT APPLICATION

(21) Application number: **85103245.8**

(22) Date of filing: **20.03.85**

(51) Int. Cl.⁴: **C 12 N 15/00**
**C 12 N 7/00, A 61 K 39/21**

(30) Priority: **26.03.84 US 593339**

(43) Date of publication of application:
**02.10.85 Bulletin 85/40**

(84) Designated Contracting States:
**AT BE CH DE FR GB IT LI LU NL SE**

(71) Applicant: **CHIRON CORPORATION**
**4560 Horton Street**
**Emeryville California 94608(US)**

(72) Inventor: **Luciw, Paul A.**
**2 Anchor Drive No. 389 Emeryville**
**California 94608(US)**

(72) Inventor: **Dina, Dino**
**1254 Washington Street San Francisco**
**California 94108(US)**

(74) Representative: **Glawe, Delfs, Moll & Partner**
**Patentanwälte**
**Rothenbaumchaussee 58 Postfach 2570**
**D-2000 Hamburg 13(DE)**

(54) LTR modified viral vaccines.

(57) Novel viral polynucleotide sequences and virus preparations recovered therefrom are provided for use as vaccines. In viruses having long terminal repeats (LTRs), where the LTRs include transcriptional regulatory functions, at least one of the LTR regions is modified to substantially decrease the activity of at least one locus involved in transcriptional regulation. Specifically, feline leukemia virus (FeLV) is modified in the LTR by deleting at least a portion of an enhancer region and the resulting modified FeLV DNA and modified virus derived therefrom shown to be infectious and capable of inducing an immune response in a mammalian host without the serious pathological symptoms normally associated with FeLV.

*E. coli* strain HB101 (pRF3X) was deposited at the American Type Culture Collection on October 14, 1983 and granted accession no. 39470.

*FIG.__4.*

# LTR MODIFIED VIRAL VACCINES

## BACKGROUND OF THE INVENTION

### Field of the Invention

Many viruses have a characteristic structure involving long terminal repeats; particularly, retroviruses are noted for this characteristic structure. Retroviruses have been associated with a wide variety of diseases, including lymphosarcomas, leukemias, thymic lymphomas, fibrosarcomas (with helper-dependent feline sarcoma virus) and non-regenerative anemias. Feline leukemia virus (FeLV), a retrovirus having three different subgroups, has also been shown to suppress the immune system of the host.

The retroviruses have been implicated in a large number of diseases among a wide variety of mammalian and avian hosts. Recently, a retrovirus ("HTLV") has been implicated with human pathology. Because of the widespread occurrence of these viruses, their pathogenicity, their detrimental and frequently fatal effect on their hosts, and the continued lack of truly effective means to combat the diseases caused by retroviruses, there remains a continuing need to develop effective methods for protecting hosts against infection.

### Description of the Prior Art

Hardy, _JAAHA_ (1981) _17_:951-980 provides a comprehensive review of the knowledge concerning feline leukemia virus and its symptomology and epidemiology. See also Sarma and Log, _Virology_ (1973) _54_:160-169 and Jarrett, Cold Spring Harbor Conf. Cell Proliferation (1981) _8_:603-611 for additional reviews especially concerning the three FeLV subgroups. Jarrett _et al._, _Int. J. Cancer_ (1975) _16_:134-141; Yohn _et al._, _Cancer Research_ (1976) _36_:646-651; Salerno, _J. Natl. Cancer Inst._ (1978) _61_:1487-1493 and Pedersen _et al._, _Am. J. Vet. Res._ (1979) _40_:1120-1126 discuss various vaccines

2

for protection against infection by FeLV. Jarrett and Russell, Int. J. Cancer (1978) 21:466-472 describe the differential cytotropism of FeLV. Lenz and Haseltine, J. Virol. (1983) 47:317-328 describe the leukemogenic determinant of a murine leukemia virus. Mullins et al., ibid. (1983) 38:688-703; Lenz, ibid. (1982) 42:519-529; Kahn et al., ibid. (1982) 41:435-448 and Bosselman et al., ibid. (1982) 44:19-31, describe various characteristics and sequences of leukemia viruses and proviruses. Robinson et al., Proc. Natl. Acad. Sci. USA (1982) 79:1225-1229 report regions of the viral genome which determine the oncogenic potential of avian leukosis viruses. Studies of transcriptional enhancers present in viral sequences may be found in Laimins et al., Proc. Natl. Acad. Sci. USA (1982) 79:6453-6457; Weiher et al, Science (1983) 219:626-631; Hampe et al., J. Virol. (1983) 45:466-472; Kelly, ibid. (1983) 45:291-298; Kriegler and Botchan, Mol. Cell. Biol. (1983) 3:325-339; Elder and Mullins, J. Virol. (1983) 46:871-880; and Luciw et al., Cell (1983) 33:705-716. Infectivity of cloned viral DNA species injected into animals has been demonstrated by Chan et al., Science (1979) 203:887-892 and Will et al., Nature (1982) 299:740-742. Unless otherwise specified and/or described, molecular cloning procedures are those indicated by Maniatis et al., Molecular Cloning, A Laboratory Manual, Cold Spring Harbor Laboratory (1982). Plasmids pKC125 and pSVgt1 are described in Mullins et al., J. Virol. (1981), 38:628-703 and Mulligan and Berg, Mol. Cell Biol. (1981) 1:449-459, respectively. See also, U.S. Patent No. 4,405,712.

## SUMMARY OF THE INVENTION

Novel methods and compositions are provided for use in vaccination of warm blooded (homoiothermic) animals, e.g. mammalian or avian hosts for protection against a wide variety of viruses having LTRs associated with transcriptional regulatory functions. Particularly, retroviruses, including oncogenic retroviruses or oncornaviruses, are modified by diminishing or destroying the transcriptional regulatory capability of at least one transcriptional regulatory function, particularly the enhancer, to produce an infectious virus or viral DNA substantially lacking the serious pathogenic symptomology or potential of the wild type. Either the modified viral DNA or virus derived from it may then be used as a vaccine for vaccinating a host against infection. Particularly, the LTR of leukemia viruses is modified by introducing a deletion into the enhancer region of the LTR which is copied in progeny virus, while removing at least that portion of the other LTR which could restore wild-type activity by recombination.

Methods and compositions are also provided for the construction of hybrid, modified viral DNA or viruses having both reduced transcriptional capability and altered immunological properties. Particularly, the modified LTRs of one retrovirus and at least the envelope gene(s) of another are combined so as to provide for reduced pathogenicity dictated by the components of the first virus but the immunogenicity of the second. Such hybrid, modified viral DNA or viruses derived therefrom may be used for vaccination of a host against the second virus.

BRIEF DESCRIPTION OF THE DRAWINGS

Figures 1 depicts the DNA sequence homology between FeLV-B and leukemogenic MCF-MuLV.

Figure 2 depicts the nucleotide sequences of the LTRs of FeLV-A, -B and -C.

Figure 3 is a schematic of the protocol for modifying the LTRs of FeLV-B.

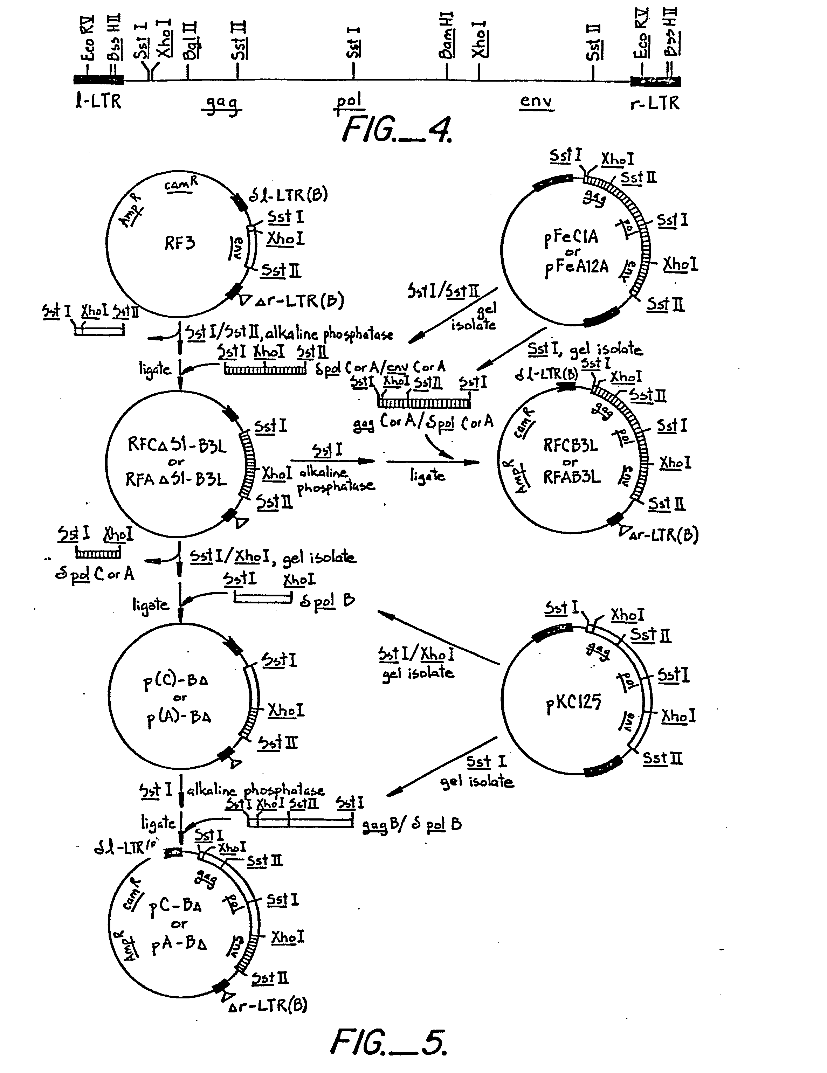

Figure 4 depicts a restriction endonuclease map of FeLV-A, -B and -C proviral DNA.

Figure 5 sets forth a schematic of the protocol for the construction of hybrid viral DNAs containing modified FeLV-B LTRs and FeLV-A or -C structural gene(s).

## DESCRIPTION OF THE SPECIFIC EMBODIMENTS

Novel polynucleotide sequences are provided of modified viruses, particularly RNA viruses, having long terminal repeats (LTRs), which viruses are of diminished infectivity and have modified LTRs where complementarity of the two LTRs of the modified virus genome is destroyed and thus does not provide for a complete wild-type LTR e.g, by recombination. Particularly, at least one of the LTRs is modified in a sequence involved in transcriptional regulation of the viral genes, which modification is not compensated for by the other LTR. The modified polynucleotide sequences may be cloned and provided as a provirus having two modified LTRs, portions of each of which subsequently serve as a template for the replication of LTR-derived sequences in progeny virus RNA genomes and subsequent formation of both LTRs that occur in cellular viral DNA.

The modified LTRs may be employed in conjunction with DNA sequences coding for proteins or fragments thereof which are immunogenic and upon expression of such polypeptides will induce an immunogenic response in a mammalian or avian host. Of particular interest are capsid proteins, envelope proteins, membrane proteins, or the like, which will provide an anamnestic response upon infection of a host with a pathogen. Other genes which may be present include the gag and pol genes, in combination with one or more env genes, which genes may encode for one or more proteins.

Of particular interest are modified retroviruses, which have single-stranded RNA genomes and are in many instances pathogenic. Of particular relevance to the subject invention is the fact that the retroviruses are characterized by having left and right

LTRs, where each of the LTRs includes sequences involved in transcriptional regulation. By modifying one or more of the sequences involved in transcriptional regulation, so as to substantially diminish but not destroy transcriptional ability of the viral DNA, either the modified provirus, e.g. as a cloned viral DNA insert, when transfected into a host, or the modified progeny virus, upon infection, e.g. by inoculation, acts as a vaccine to protect the host against infection from a wild-type virus, or other pathogen sharing immunogenic properties with the modified retrovirus, either because of natural cross-reactivity with the retrovirus having a modified LTR or by insertion of or substitution with exogenous DNA.

The life cycle of the retrovirus results in a variety of structures. The viral RNA genome has different sequences at the 5'- and 3'-termini referred to as U5 and U3, respectively. Upon reverse transcription into DNA the termini result in the left and right long terminal repeats (1- and r-LTR) which are combinations of U3 and U5 in that order at each terminus. This cellular viral DNA may then become integrated into the host genome to provide the proviral DNA.

Since DNA is more readily manipulated, the two LTR's are modified so as to be non-complementary, rather than directly modifying U3 and/or U5 which would provide the same result. Thus, the virus is attenuated or disarmed by providing at each stage of its life cycle a defective transcriptional regulatory system.

When referring to modification, the modification includes removal of a portion of or all of the enhancer region of the LTR. Generally at least one base will be involved, more usually at least two bases, preferably at least about 5 bases, and more preferably 20 or more bases, and usually not more than 250 bases, more usually not more than 150 bases. The modification results in diminution of the transcriptional activity

6

resulting from the LTR, while substantially diminishing the replication capability of the virus, as well as its pathogenicity. Usually, replication will be diminished under comparable circumstances of growth between the wild-type virus and the modified virus by a factor of at least 2, more usually by a factor of at least about 5.

The modification to the viral DNA may involve a deletion, substitution, inversion, insertion, or the like. The significant factor is that the modification should provide an extremely low, preferably no, possibility of reversion to wild-type and the other LTR must be changed in a manner which inhibits complementarity by such other LTR to restore wild-type transcriptional activity by recombination.

These modifications will involve any of a large number of retroviruses known to be pathogenic to mammalian or avian hosts. Included among these retroviruses are human T cell leukemia virus (HTLV), feline leukemia virus (FeLV), murine leukemia virus (MuLV), bovine leukemia virus (BLV), feline sarcoma virus (FeSV), murine sarcoma virus (MuSV), simian leukemia virus (SLV), simian sarcoma virus (SSV), avian leukosis virus (ALV), equine infectious anemia virus (EIAV), or the like. These retroviruses also referred to as oncornaviruses, are the primary focus of the subject invention. Nevertheless, other viruses which share characteristics comparable to the subject viruses, and which may be modified in a manner analogous to the subject viruses may also find use.

The long terminal redundant or repeat sequences of the oncornaviruses or retroviruses will generally range from about 300 to 1200 base pairs (bp). Illustrative of these long terminal repeats is the LTR of feline leukemia viruses which are about 475-500 bases per strand with the provirus LTRs being 484 for subgroup A and 488 for subgroup C, but being 482 for the subgroup

B genome. The LTRs will normally include within their sequence a number of transcriptional regulatory sequences for initiation, processing etc., such as a promoter, enhancer, TATA box, CAAT box, cap sequence, polyadenylation processing signal, and terminator. The enhancer will generally be of about 50 to 100bp and will usually be found about 100 to 150bp upstream from the TATA box. The CAAT box will generally be about 50bp upstream from the TATA box. The viral RNA genome will usually have two LTR-related regions (U3 and U5), while the cellular viral DNA and integrated provirus will usually repeat the same LTR derived therefrom by reverse transcription (RNA → DNA) and replication.

The oncornaviruses have numerous species, subspecies, strains and subgroups, which may differ in a variety of ways and may result in different immunogenicity. Therefore, depending upon the particular oncornavirus involved, one or more subgroups of the oncornavirus may be necessary to provide for complete protection in a specific mammalian host. For example, with FeLV which has at least three subgroups, A, B and C, it may be sufficient to provide for immunization with the modified subgroup B or a combination of two or more subgroups may be necessary.

In accordance with the subject invention, a DNA sequence is provided which retains the genetic functions of the wild-type virus essential for limited replication and infectivity, while being modified so as to have a substantially reduced pathogenicity and rate of replication in a host. This can be achieved by a variety of strategies. An exemplary strategy is to diminish or destroy the enhancer function in one LTR, particularly the right LTR, while also destroying the ability of the left LTR to provide complementary sequences to the lost enhancer structure, to prevent restoration of the wild-type enhancer function by recombination.

Diminution of the enhancer function can be achieved by a mutation, inversion, insertion, deletion, or combinations thereof preferably by at least a deletion. Deletions can be conveniently introduced where a restriction endonuclease site is present. This may involve insertion of the viral DNA into a convenient vector for amplification in a suitable host and purification, followed by molecular genetic manipulation of the virus in the LTR region. Desirably, the restriction endonuclease site in the LTR used for genetic manipulation should be unique to the LTR regions, although partial digestions can be employed with lower efficiency. A deletion may then be introduced by treatment with a double-strand-specific exonuclease.

While the left and right LTRs may be treated the same, they may also be treated differently. To that extent, the left and right LTRs may be separated for modification into different plasmids with appropriate flanking regions of the LTRs, so that the two portions of the virus may be subsequently reassembled to restore the viral DNA structure to provide for the reduced replication and infectious function. To avoid complementarity and thus prevent recombination dependent upon DNA sequence homology, the other LTR may be truncated, conveniently from the same restriction endonuclease site at which the above deletion was introduced in the first LTR.

Other mechanisms exist for modifying the LTRs, such as primer repair, which can provide for replication from a particular site in the LTR and loss of the LTR upstream from that site, in vitro mutagenesis, which can provide for deletions, insertions or mutations, use of transposons to provide for deletions, or the like. The particular mechanism used to destroy complementarity of the modified regions in the two modified LTRs present in the cloned provirus, portions of each of which are to serve as a template and be transcribed, replicated and retained in the progeny virus or cellular provirus, is not an essential aspect of this invention.

Experience with the LTRs of one retrovirus can be translated to other retroviruses. For example, the three subgroups of feline leukemia virus and several strains of murine leukemia virus have a high degree of homology in the LTR. The MCF-MuLV has an enhancer region that is 75bp long, which can be aligned with the enhancer region of FeLV-B, where the 67bp enhancer segment of the FeLV shares 56bp with the MCF-MuLV. Thus, a comparison of the regulatory sequences from one retrovirus with those of others can be used for the determination of analogous regulatory sequences in different retroviruses by nucleotide sequencing and the like.

Once the appropriately modified LTR has been manipulated and cloned, the plasmid containing the viral DNA with a modified LTR may be used as a provirus for either transfection of tissue culture host cells and passaging or introduction into an animal via inoculation. From transfected cells showing virus production, which can be determined by any convenient assay, e.g. an assay for reverse transcriptase activity, the viruses may be recovered by any convenient procedure. Alternatively, the virus may be harvested and used for infection of a host and the host bled to produce additional virus and/or antibody.

The subject viral DNA compositions and virus derived therefrom find use as vaccines. Hosts may be inoculated by any convenient means with a sufficient amount of either the modified, cloned proviral DNA or the virus to provide an immune response. The amount of virus will be from about $10^4$ to about $5 \times 10^6$, usually $5 \times 10^5$, focus-forming units/kg host while the amount of viral DNA will be about 50 to 1000μg DNA/kg host. The viral DNA, provirus or virus may be in any convenient physiologically acceptable medium, e.g. sterile water, PBS, growth medium, or the like.  Usually, the dosage volume will be 0.5 to 2ml and administered by injection subcutaneously, intramuscularly, intraperitoneally, intravenously, or the like.  These vaccines may be administered to previously primed hosts.  One or more booster injections may be employed at weekly to 6 week, usually 2-4 week, intervals.

Hosts of interest include domestic mammalian and avian species such as murine, feline, bovine, equine and canine hosts, chickens and other poultry, as well as primates.

The following examples are offered by way of illustration and not by way of limitation.

## EXPERIMENTAL

The following materials and methods were used for the various manipulations.

Restriction Enzyme Buffers and Conditions

---

TABLE 1

---

| Restriction Enzymes | Buffer (10X) |
|---|---|
| (Low salt) | 100mM NaCl, 100mM MgCl$_2$, |
| ApaI, ClaI, BalI, HpaI, | 100mM Tris, pH 7.5 |
| KpnI, NciI, SmaI, SstII | |
| (SacII), PaeR7 | |
| | |
| (Medium salt) | 500mM NaCl, 100mM MgCl$_2$, |
| AccI, AvaI, AvaII, BglI, | 100mM Tris, pH 7.5 |
| BglII, BssHII, BstEII, EcoRI, | |
| HindIII, HincII, MluI, NaeI, | |
| NarI, NcoI, NdeI, NruI, PstI, | |
| PvuII, SstI (SacI), SphI, | |
| StuI, Tht111I, XmnI | |
| | |
| (High salt) | 1.5M NaCl, 100mM MgCl$_2$, |
| BamHI, EcoRV, MstII, SalI, | 100mM Tris, pH 7.5 |
| XhoI, XbaI | |

25µl total reaction volume, 2 units of restriction enzyme per µg DNA at 37°C, except BstEII and Tht111I, 60°C, BssHII, 50°C.

---

Ligation

One µg DNA in a total volume of 100µl of buffer (10mM Tris, pH 7.5, 10mM MgCl$_2$, 2mM ATP, 5mM DTT) and 1µl T4 ligase (New England Biolabs, approximately 400 units), incubated at 4°C for 4-6hr. For blunt end ligations, include 1.5mM spermidine in the reaction mixture and ligate for a total of 14-24hr.

0156299

## Klenow Procedure for Making Blunt Ends

A reaction volume of 50μl of a buffer (6mM Tris, pH 7.5, 6mM $MgCl_2$, 1mM DTT, 50mM NaCl) containing 50μM each of the four dNTPs, 5 units of DNA polymerase I klenow fragment (New England Biolabs) and 1-10μg DNA, the mixture incubated at 37°C for 30min and stopped by extracting with phenol/chloroform.

## Bal31 Deletions

After complete digestion of about 20μg of DNA by the appropriate restriction enzyme, the enzyme is inactivated by heating at 68°C for 5min, followed by the addition of 5x stock buffer (2.5M NaCl, 25mM $CaCl_2$, 25mM $MgCl_2$, 100mM Tris, pH 7.5) and water to a volume of about 200μl (final concentrations: 500mM NaCl, 5mM $CaCl_2$, 5mM $MgCl_2$, 20 mM Tris, pH 7.5). To the mixture is added one unit of Bal31 and aliquots are taken at different time intervals and the reaction stopped by phenol/chloroform extraction, followed by chloroform extraction and then ethanol precipitation.

For ligation of the two blunt ends, the alcohol-precipitated, resected DNA is centrifuged, the pellet washed one time with ethanol, and then resuspended in 83μl of water and 5μl each of 200mM Tris-200mM $MgCl_2$, pH 7.5, 40mM ATP, 100mM DTT and 2μl 100mM spermidine added.

## Plasmid Amplification and Isolation

To 1ml of an overnight saturated bacterial culture is added 100ml of an amplification medium (20X M9 mix, 5ml; 10X M9 salts, 10ml; L-broth, 10ml; 1% B1, 0.1ml; 20% CAA, 0.5ml; 1% Leu, 1ml; 1% Pro, 1ml; 2% Amp/DMSO, 0.2ml) and cells grown to O.D. 650=1 in a 37°C-airshaker (about 4hr). To the mixture is then added 10ml L-broth; 0.5ml 20% CAA and 1ml of 50% glucose and the cells grown for an additional 30min. To the mixture is then added 0.5ml of a freshly prepared solution of 50mg/ml chloramphenicol in 100% ethanol and the plasmid DNA amplified overnight.

The culture is centrifuged in a 250ml plastic bottle (Beckman), 3.5K rpm/20min in a Beckman J-6B, the pellet suspended in 10ml of 0.15M NaCl, 50mM Tris, pH 7.5 and centrifuged as described before. The pellet is then resuspended in 1ml G.E.T. (25mM Tris, pH 8, 10mM EDTA, 50mM glucose) and an additional 1ml of G.E.T. added containing 4mg/ml of lysozyme, and the mixture cooled on ice for 45min. To the mixture is then added 4ml of 0.2N NaOH + 1% SDS followed by gentle mixing and standing on ice for 15min. To the mixture is then added 3ml of a 3M solution of potassium acetate, pH 4.8, and after cooling on ice for 1hr, the mixture is centrifuged at 10K rpm/20min in a Beckman J2-21 (rotor JS-13). To the supernatant is added an equal volume of isopropanol, the mixture allowed to stand at room temperature for 5min, followed by centrifugation at 10K rpm/20min. The resulting pellet is suspended in 1ml T.E. and 5µl RNAse A (10mg/ml in 50mM sodium acetate, pH 4.8; boiled 10min) added and the mixture allowed to stand at room temperature for 20min. To the mixture is then added 0.5ml of 30% PEG 8000/1.5M NaCl and it is then cooled on ice for 30min, followed by centrifugation at 10K rpm/20min. The pellet is then redissolved in 0.4ml T.E., transferred to a 1.5ml microfuge tube and extracted two times with phenol/chloroform followed by one time with chloroform. The aqueous phase is then split into two 400µl aliquots in two tubes and 3 volumes ethanol added to each. The alcoholic and aqueous phases are then mixed at which time a thread-like DNA precipitate occurs. After spinning in a microfuge for 5min, the DNA is ethanol rinsed and dried in a Speedvac. The combined pellets may then be dissolved in water or T.E., normally yielding about 0.3-1.0mg.

## Bacterial Transformation

To 100μl of competent E. coli HB101 cells is added 100μl DNA sample (about 1μg DNA), the mixture incubated at 4° for 20min, 37° for 2min and 15min at room temperature. To the mixture is then added 1ml L-broth and the mixture is shaken at 37°C for 60-90min, followed by plating on L-agar under selective conditions.

## Plasmid Selection

Unless otherwise indicated (e.g., drug resistance), plasmids (i.e., bacterial host colonies) are selected by transfer to nitrocellulose filters and use of appropriate radiolabeled (in vivo or enzymatically in vitro) probe(s), e.g., a gel isolated restriction enzyme fragment derived from FeLV-B DNA, and/or use of minipreps followed by sizing and/or restriction enzyme mapping using gel electrophoresis, e.g., to test for orientation. Also, in many cases when appropriate, one of the DNA fragments to be enzymatically joined, usually the vector, is treated with alkaline phosphatase before ligase to help insure proper ligation, i.e., prevent recircularization of the vector itself without fragment insertion or the occurrence of tandem inserts, etc.

## Reverse Transcriptase Assay

See Rosenberg and Haseltine, Virology (1980) 102:240-244.

## Preparation of Modified FeLV-B (pRF1,2 and 3X)

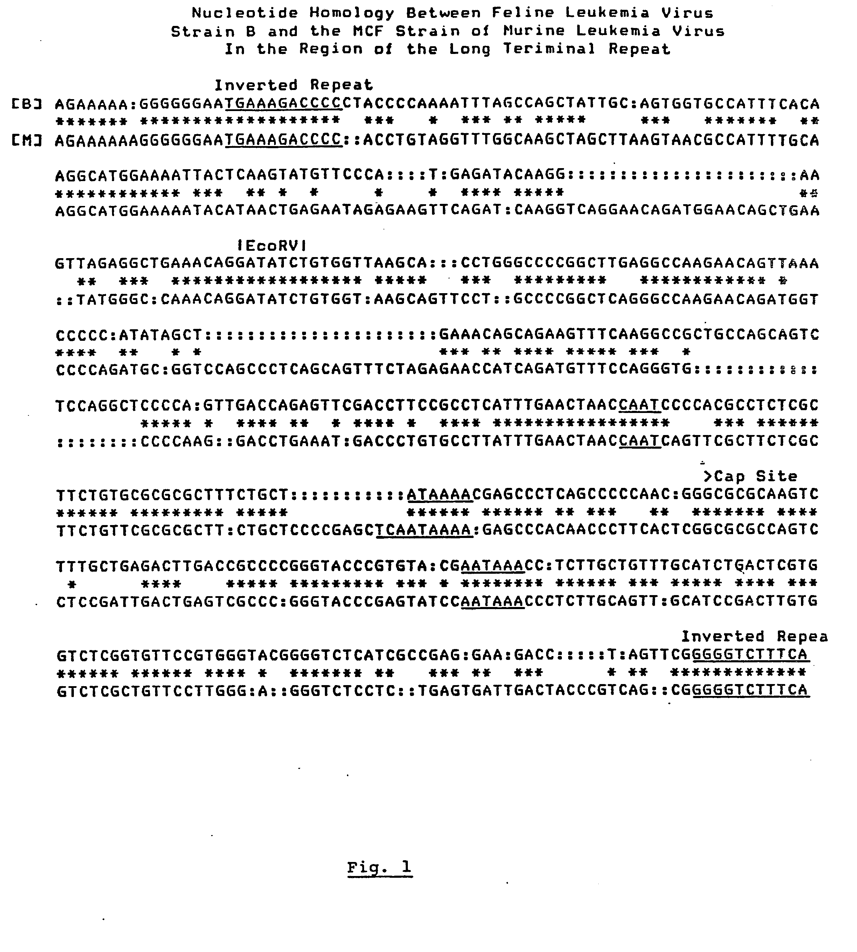

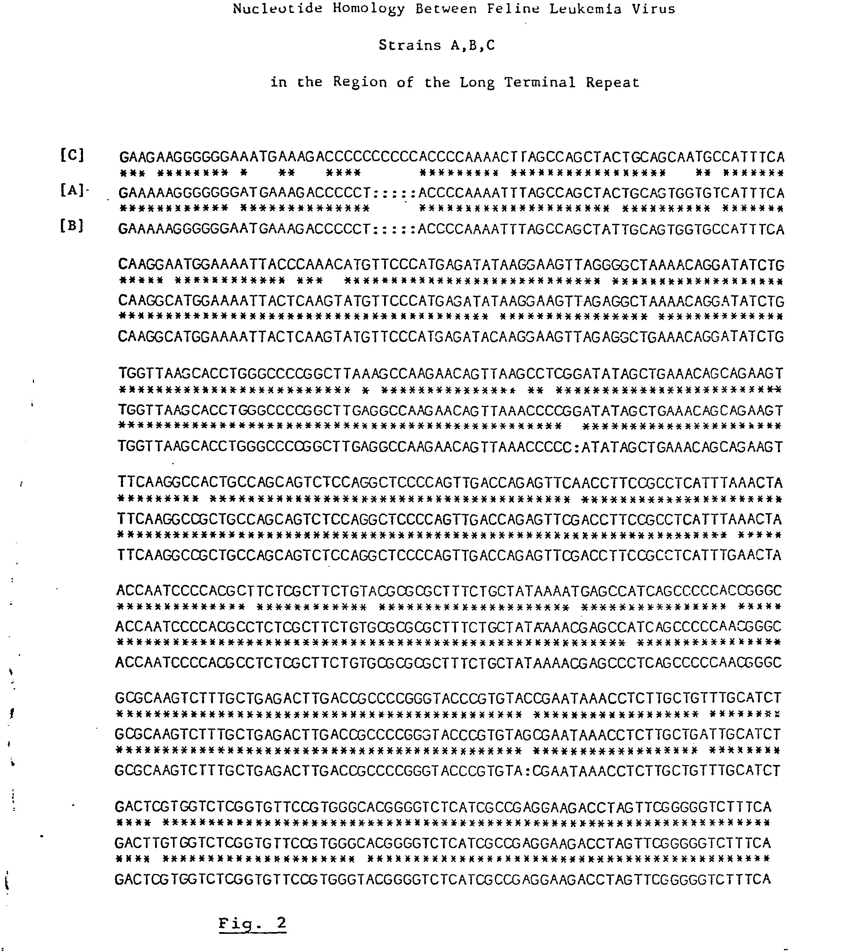

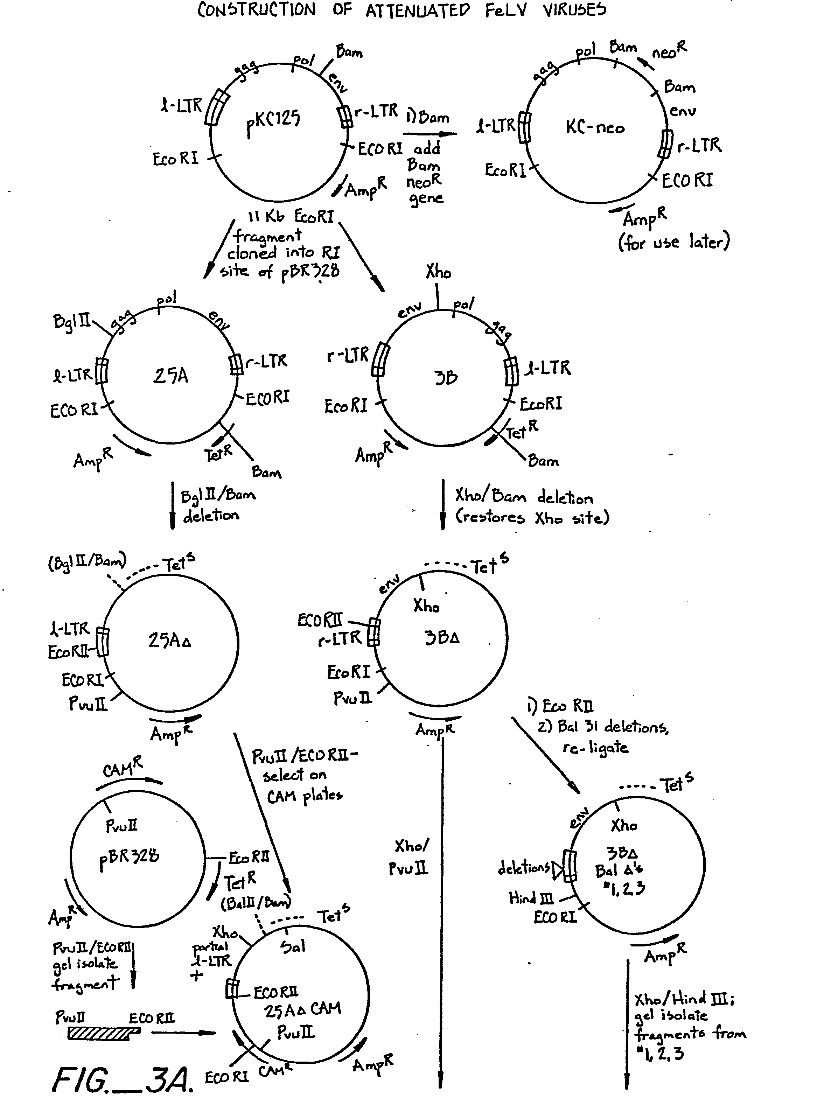

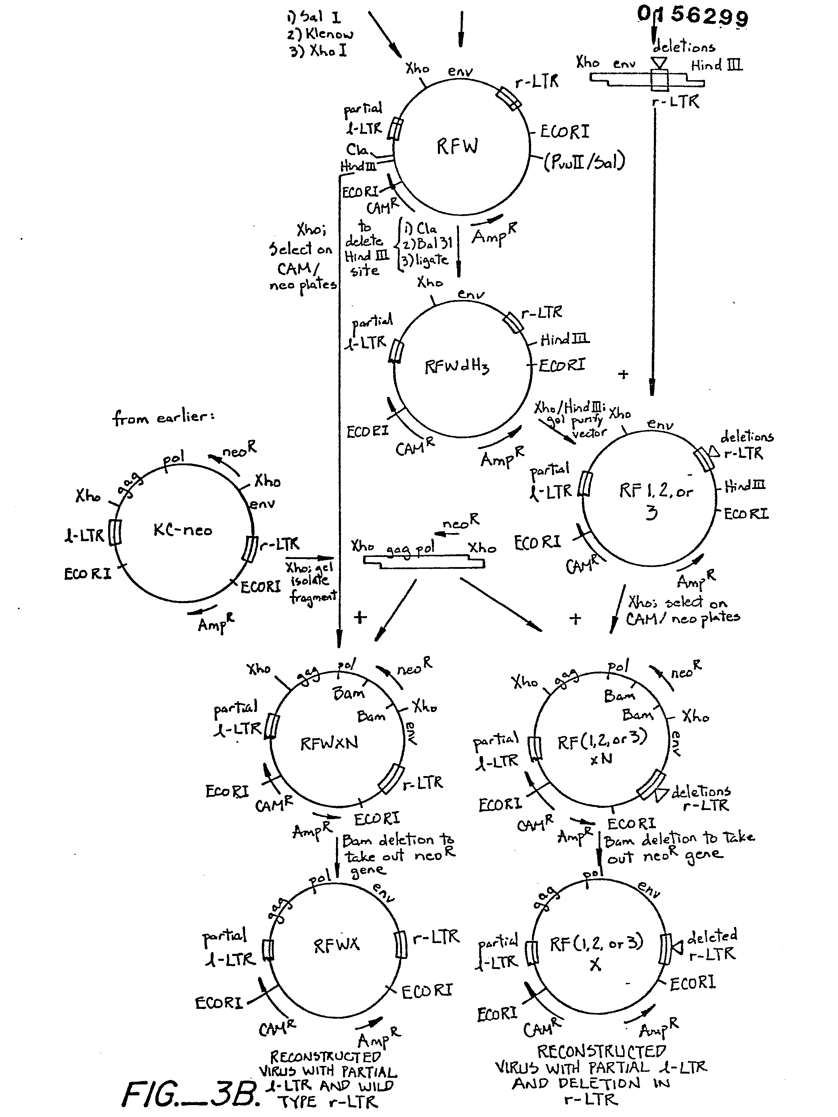

The DNA sequence homology between FeLV and MCF-MuLV are set forth in Figure 1. The LTR nucleotide sequences of FeLV-A, -B and -C are set forth in Figure 2. A schematic of the protocol for this modification is set forth in Figure 3.

In order to prepare the modified FeLV-B, pKC125 (obtained from Dr. James Casey, Louisiana State University) was employed, where an 11kb EcoRI fragment

containing the complete genome of FeLV-B, with single copies of the left and right-LTR was inserted into the EcoRI site of pBR328. The plasmid has an ampicillin resistance gene. The plasmid was used in two ways: (1) to provide the right-LTR (r-LTR) with deletions at the EcoRV site; and (2) to provide a truncated left-LTR (l-LTR), from which all the sequences upstream from the EcoRV site had been removed.

(1) An approximately 11kb EcoRI fragment from pKC125 was inserted into the EcoRI site of pBR328 to provide two plasmids having different orientations of the EcoRI fragment, p3B having the orientation from right- to left-LTR in the opposite direction of the orientation of the ampicillin resistance gene, while p25A had the orientation from right- to left-LTR in the same direction as the orientation of the ampicillin resistance gene, with the l-LTR proximal to the 5'-end of the $amp^R$ gene. p3B was used for introducing deletions into the r-LTR. To isolate the r-LTR and to remove all EcoRV sites in the plasmid except the one in the LTR, p3B was digested with a mixture of XhoI and BamHI. The resulting 4bp 5'-overhanging ends were filled in with reverse transcriptase or the klenow fragment of DNA polymerase I. These molecules were circularized with T4 DNA ligase and cloned to provide p3BΔ, where the recircularization restores the XhoI site. The resulting plasmid is ampicillin resistant and tetracycline-sensitive, as compared to the precursor plasmid which is $amp^R$ and $tet^R$. p3bΔ was then digested with EcoRV and resected with Bal31 for different periods of time to provide deletions ranging from something under 50bp to a plasmid having a deletion of about 125-150bp. These three deletions will be referred to as 1, 2 or 3, ranging from the smallest to the largest deletion in that order. Sequencing showed 1 to have a 22bp deletion at position -234 to -212; 2, a 57bp deletion at -251 to -194; and 3, a 132bp deletion at -286 to -154, where the first base of the r-LTR is defined as -338. See Figure 1. The resulting plasmids had a HindIII-XhoI fragment which included the r-LTR with the different deletions and extended through the env gene.

(2) In order to avoid recombination resulting in a wild-type LTR, the l-LTR was now modified. p25A was completely digested with BglII and BamHI which resulted in two fragments, one fragment having the l-LTR, the amp$^R$ gene and an inactivated tet gene. This DNA was circularized with T4 DNA ligase, transformed into E. coli, recovered by selection for amp$^R$ and tet$^S$ and designated p25AΔ. p25AΔ was then digested with PvuII and EcoRV to provide a fragment where the l-LTR was reduced in size by removing U3-LTR sequences upstream from the EcoRV site. This fragment was joined by ligation with T4 DNA ligase to a PvuII/EcoRV fragment isolated from pBR328, which fragment had an intact chloramphenicol gene. The resulting plasmid p25AΔcam was cam$^R$, amp$^R$ and tet$^S$. The l-LTR now lacks a portion of the enhancer region.

p25AΔcam was totally digested with SalI, the overhang filled in using the Klenow DNA polymerase I fragment, followed by complete digestion with XhoI. p3BΔ was totally digested with XhoI and PvuII to provide a fragment containing an intact r-LTR and the env gene. The two fragments were joined to provide plasmid RFW which was cam$^R$ and amp$^R$ and included the partial l-LTR.

In order to delete a HindIII site adjacent the partial l-LTR, plasmid RFW was digested with ClaI, resected with Bal31 and ligated to provide plasmid RFWdH3, which lacked the HindIII site adjacent the partial l-LTR. Plasmid RFWdH3 was then completely digested with XhoI and HindIII, and gel purified. The plasmids p3BΔ1, 2 and 3 and the fragments containing the r-LTR with the different deletions were isolated. The fragments from p3BΔ1, 2 and 3 were each combined with the fragment from plasmid RFWdH3 to provide plasmids RF1, 2

or 3, which were cam$^R$, amp$^R$ and included the partial l-LTR, the deleted r-LTR and the env gene.

The bacterial neo$^R$ gene was excised from pNeo (Bethesda Research Laboratories, Gaithersburg, MD) as a HindIII/EcoRI fragment and cloned into the HindIII/EcoRI digested, SV40 expression vector plasmid, pSVgtl (available from Dr. Paul Berg, Stanford University) for use as a selectable marker in tissue culture cells (Southern and Berg, J. Mol. Applied Genet. (1982) 1:327-341). This plasmid contains convenient BamHI restriction sites flanking the neo$^R$ gene allowing its excision as a Bam fragment.

In order to restore the other structural genes necessary for infectivity, the plasmid pKC125 was completely digested with BamHI and the Bam fragment having the neomycin resistance gene inserted into the Bam site. This plasmid KC-neo was completely digested with XhoI and a fragment isolated by gel electrophoresis which included the gag and pol FeLV-B genes and the neo$^R$ gene. This Xho fragment was then inserted into the Xho site of plasmids RF1, 2 or 3 to provide plasmids RF(1, 2 or 3)XN, where the FeLV-B virus had the neo$^R$ gene on a BamHI fragment inserted between the pol and env genes while having the modified LTRs in their proper orientation. The subject plasmids were selected on cam/neo plates. The neo$^R$ gene was then removed by BamHI digestion and selection for cam$^R$ neo$^S$ to provide plasmids RF(1, 2 or 3)X, which now had the reconstructed virus with a partial l-LTR and r-LTR containing the previously indicated deletions.

In order to provide a comparison, a plasmid was constructed having FeLV-B virus with a partial l-LTR and an intact r-LTR. This construction was derived from the plasmid RFW by digestion with XhoI and combining and ligating with the XhoI fragment from plasmid KC-neo, which contains the gag and pol genes, as well as the neo$^R$ gene on a BamHI fragment. The resulting

(RFWXN) plasmid was selected on cam/neo plates, followed by digestion with BamHI to remove the $neo^R$ gene to provide plasmid RFWX, which has an intact FeLV virus except for the partial 1-LTR.

## Cells and Tissue Culture

The following cell lines were employed with their source or reference being indicated Lu-1(cat, AK-D) and Tg (cat, Fc3Tg) (A.T.C.C.); RD-4 (human), D-17 (dog) and Cos-1 (monkey) cell lines (Tissue culture facility at U.C.S.F.). Cells were propagated in Dulbecco's modified Eagles Medium with 10% fetal calf serum (FCS). Cell cultures were incubated at 37°C, in 95% air, 5% $CO_2$.

For passaging cells were removed from tissue culture dishes by treating the monolayer with a small volume of 0.25% trypsin in STV (saline/trypsin/versene) for 5-10min at room temperature. Cell stacks were frozen at -85°C in 50% FCS, 10% DMSO growth medium. STV is (per liter of double-distilled water): 8.00g NaCl, 0.4g KCl, 1.00g glucose, 0.58g $NaHCO_3$, 2.5g trypsin (Difco 1:250), 0.2g versene (EDTA) (Gibco), 0.0045g phenol red; pH adjusted to 7.0-7.1 and sterile-filtered before use.

Two RD-4 cell lines, one infected with FeLV-A/Glasgow-1 and the other with FeLV-C/Sarma strains were obtained from Dr. James Casey of Louisiana State University.

## Cocultivation to Rescue Virus

$2x10^6$ Cos-1 cells harboring FeLV-A proviruses were mixed with $2x10^6$ Lu-1 cat cells and seeded in 100mm tissue culture dishes in growth medium. After reaching confluency in 5 days, the monolayers were resuspended in 0.25% trypsin-STV and $5x10^6$ cells were seeded in fresh medium in 100mm plates and allowed to reach confluency. After 5 days the cells were passaged once more, grown for 5 days, and the medium was then harvested. This medium was used to infect fresh Lu-1 cells and growth of virus was determined by the reverse transcriptase assay. If the culture were positive, virus was harvested and frozen at -85°C.

## Virus Infection of Tissue Culture Cells

One ml of medium (virus harvest) to be tested for virus was added to a 60mm dish containing 500,000 cells in 4ml of growth medium. After 6hr at 37°C in an incubator (95% air, 5% $CO_2$) the medium was replaced with fresh growth medium and the dishes returned to the tissue culture incubator. Infected cells were passaged at approximately 5 day intervals. For each passage, cells were diluted 1:10 and seeded in new dishes with fresh medium.

## Virus Harvests

Infected cells were grown on tissue culture dishes until they approached about 80% saturation (as judged by visual inspection with a microscope). Fresh growth medium was applied and cells were incubated at 37°C for 12hr. This medium was collected and filtered through a 0.22µ filter to remove cells and cell debris. In some cases, a large amount of medium was collected and centrifuged at 5,000rpm for 10min at 4°C to pellet cells and cell debris (clarified virus). The supernate was transferred to a 36ml polyallomer tube and centrifuged in an ultracentrifuge at 25,000rpm for 1hr at 4°C in a SW28 rotor. The supernate was decanted and the pellet (containing virus) was resuspended in 1ml of growth medium. Medium from uninfected cells was similarly prepared for mock-infected controls.

## DNA Transfection

Co-precipitates of DNA and calcium were prepared according to modifications of the method of Graham and Van der Eb, _Virology_ (1973) <u>52</u>:456. The co-precipitates were made in Hepes buffered saline (HBS; 16g NaCl, 0.74g KCl, 0.39g $Na_2HPO_4 \cdot 7H_2O$, 1g dextrose, 5g Hepes, pH adjusted to 7.05). A total mass of 20µg of DNA (composed of 18µg of salmon sperm carrier DNA and 2µg of FeLV-plasmid DNA) was dissolved in 0.5ml of 250mM $CaCl_2$. To this solution was added dropwise, 0.5ml of HBS and the solution mixed by bubbling a stream of air. To one dish (60mm) of 500,000 cells in 5ml growth medium was added 0.5ml of the co-precipitate. The dishes were incubated 6hr at 37°C (95% air, 5% $CO_2$); the medium was then removed by aspiration, and the cell monolayers were treated for 4min at room temperature with 1ml of a 15% glycerol solution in HBS. The glycerol solution was then replaced with 5ml of growth medium and the monolayers placed in a cell incubator. After 4-5 days, the cells were passaged by diluting cells from the confluent dish 1:10 in a new dish with fresh medium. The cells were similarly passaged 3 additional times, and the medium was assayed for reverse transcriptase activity.

Animals

Eight week old kittens were obtained from U.C. Davis (Dr. Quentin Rogers, School of Veterinary Medicine and Dr. Murray Gardner, School of Medicine). Each cat had an identification number tatooed on its left ear. Pairs of kittens were maintained in steel cages at the animal facility in the Naval Biosciences Lab, Oakland, California (Dr. David Kingsbury). The animals were free-fed with Purina cat chow.

Inoculation of Cats

Cats were inoculated with either proviral DNA, i.e., plasmids containing the modified virus genome, or virus preparations derived therefrom produced by transfection of tissue culture cells. In the latter case cats were inoculated with 1.0ml of virus containing at least $10^5$ focus-forming units (either clarified medium or pelleted virus); 0.5ml was given (26-gauge needle on a 1ml tuberculin syringe) subcutaneously in the shoulder and 0.5ml was given intramuscularly in the hind leg flank. Control cats were similarly inoculated with mock-infected medium. In the former case, cats were inoculated with 100µg of viral DNA within a plasmid resuspended in 1.0ml of PBS; 0.5ml was injected subcutaneously as above and 0.5ml was also injected intravenously in the right foreleg vein.

Serum Collection from Cats

Cats were shaved in the neck area to expose the jugular vein. A 22-gauge needle on a 5ml syringe was used to draw 2 to 3ml of blood. Whole blood was allowed to stand at room temperature for 4hr to clot and then spun at 5,000rpm for 20min. The supernate (serum) was withdrawn with a pipet and stored at -70°C. Cats were bled prior to the first inoculation and at various intervals thereafter.

Recovery and Characterization of Infectious Viruses from Plasmids Containing Proviruses

To recover infectious viruses, the reconstructed plasmids were transfected into several lines of tissue culture cells permissive for FeLV-B. After approximately 4 weeks of serial propagation, each transfected culture was tested for virus production by assaying for reverse transcriptase activity of the tissue culture medium. All cultures transfected with plasmids containing the FeLV-B proviruses, RFWX, RF1X, RF2X and RF3X were positive. A control culture that received pBR328 DNA was negative.

Virus was harvested by collecting medium, which was used to infect fresh lines of permissive cells. These infected cells were serially passaged for four weeks, ensuring that every cell in each culture was infected. To verify the structure of proviral DNA, high molecular weight DNA was prepared from the infected cell cultures by phenol-chloroform extraction, digested with restriction endonucleases and analyzed by the Southern blotting procedure. For hybridization, radiolabeled DNA probes representative of the FeLV-B provirus were prepared. Digestion with a mixture of KpnI and SacII of DNA from cells infected with wild-type virus (derived from proviral plasmid RFWX) showed the expected pattern, including a 1.0kb DNA fragment that encompasses the U3 portion of the wild-type virus LTR. Cells infected with virus derived from proviral plasmid RF3X share the same DNA fragments except that the 1kb DNA fragment is absent; instead, a new 0.9kb DNA fragment is observed. Digestion of the cellular DNA samples with EcoRV shows that wild-type (RFWX-derived) cellular provirus has EcoRV sites in both LTRs, whereas the ΔRV3 (RF3X-derived) cellular provirus has only the internal EcoRV sites and no EcoRV sites in either LTR. Thus, the integrated cellular provirus recovered from the reconstructed RF3X proviral plasmid and by inference the viral RNA genome retain the deletion in the LTR or LTR-derived region (U3), respectively.

The recovered viruses were used to infect a variety of tissue culture cells and replication was measured by assaying for virion-associated reverse transcriptase in the growth medium. The following Table 2 shows that the viruses containing the deletions grow less well than wild-type virus.

TABLE 2:  Virus replication in tissue culture cells measured
by virion reverse transcriptase activity.

| | | reverse transcriptase activity $(\times 10^3)$ | | | |
|---|---|---|---|---|---|
| Cell line | virus strain | RFWX | RF1X | RF2X | RF3X |
| Cat (Lu-1) | | 53 | 23 | 28 | 16 |
| Dog (D17) | | 24 | 4 | 7 | 2.2 |
| Human (RD4) | | 34 | 17 | 13 | 8 |

Vaccination

Cats were either inoculated with virus
preparations or injected with proviral plasmid DNAs.
Four cats were inoculated with FeLV-B wild-type virus.
Two cats were inoculated with ΔRV3 virus (derived from
the RF3X proviral plasmid), and two cats were inoculated
with concentrated ΔRV3 virus.  Four other cats were
mock inoculated with tissue culture medium of uninfected
cat cells.  Two additional groups of four cats each
were each injected with either control proviral plasmid
RFWX or proviral plasmid RF3X modified in both LTRs.
Immune responses in these animals were measured by an
ELISA test, which detects the presence of antibodies to
viral proteins.  All cats inoculated with the virus
preparations made significant levels of anti-viral antibodies and approximately one half of those injected
with viral DNA did so.  Over a period of five months,
no clinical disease systems have been noticed in any of
the animals.

Characterization of the antibodies from the
cats showed that the anti-viral antibodies did not react
with denatured virion proteins on a "Western" gel,
whereas, rabbit and mouse anti-viral antibodies did
react with denatured viral proteins in the same analysis.
The cat anti-viral antibodies from the inoculated cats
were able to immunoprecipitate $I^{125}$-labeled virion

proteins.  Different extents of reaction with gp70 were observed with different cat antibodies.

The results indicate that cats are able to raise a humoral immune response predominately to the native conformation of the viral envelope protein.

Cloning of FeLV-A and FeLV-C Proviruses with Enhancer Deletions

High molecular weight whole cell DNA from the human RD-4 cell lines infected with FeLV-A and FeLV-C (vide supra) was prepared by phenol-chloroform extraction and a sample of each digested with various restriction endonucleases and analyzed by Southern blotting with a $^{32}$P-radiolabeled probe specific to FeLV sequences: the BssHII fragment of FeLV-B DNA (pRFWX) which comprises almost the entire proviral genome since this enzyme cleaves twice in each LTR only. EcoRI restriction sites were not detected in the proviral DNA in either cell line; therefore, the whole cell DNA preparations isolated from each were digested to completion with EcoRI, centrifuged in sucrose gradients and fractions corresponding to 8-15kb were pooled, dialyzed and concentrated by ethanol precipitation. The bacteriophage λ derivative cloning vector, EMBL-4 (see Karn et al., Methods Enzymol. (1983) 101:3-19) was digested to completion with a mixture of EcoRI, BamHI and SalI restriction enzymes and the DNA then deproteinized by phenol-chloroform extraction, precipitated with cold ethanol and resuspended in ligation buffer.  (The brief alcohol precipitation selectively recovers large DNA fragments (e.g., phage arms) while the small linker DNA is retained in solution; SalI/SalI filler fragments are not incorporated into the construct during subsequent ligation.)  The EMBL-4 phage DNA and EcoRI digest of cellular DNA are mixed and ligated, and the resultant recombinant phage genomes packaged in vitro.  After phage infection of λ-sensitive E. coli,

phage plaques were transferred to nitrocellulose filters, DNA was fixed and the filters were screened with FeLV-specific radiolabeled probe as above. Positive plaques, consisting of phage containing the entire FeLV-A or C proviral DNAs, together with flanking human (RD-4 cell) DNA, were recovered.

Plasmids pFeClA and pFeA12A, were then obtained by subcloning the provirus FeLV-C or A, respectively, from EMBL-4 into pBR328. In the case of provirus A, it was inserted into the SstI/EcoRI site of the cam$^R$ gene, while in the case of C, the provirus was inserted into the EcoRI site of the cam$^R$ gene, with the resulting plasmids being cam$^S$, amp$^R$ and tet$^R$.

Two strategies were then implemented for the construction of hybrid retrovirus proviral plasmids containing the structural gene(s) of either FeLV-A or C and the previously modified LTRs of FeLV-B. (See Figure 5 for a schematic of the protocol for these constructions.) In the first method, plasmid RF3 was completely digested with SstI and SstII and treated with alkaline phosphatase. pFeClA and pFeA12A were digested with SstI and SstII to provide fragments (gel isolated) including a portion of the pol gene, and the env gene. The fragments were then inserted into the larger SstI/SstII fragment from RF3 having the amp and cam genes, as well as the partial l-LTR and deleted r-LTR, to provide plasmids RFCΔS1-B3L or RFAΔS1-B3L (selected using an appropriate probe for the insert). These plasmids were then digested with SstI, treated with alkaline phosphatase and an SstI fragment (gel isolated) containing the gag gene and a portion of the pol gene from pFeClA and pFeA12A were each inserted to provide plasmids RFCB3L and RFAB3L (probe-selected), which contain the intact structural genes of FeLV-C and FeLV-A, respectively, with the partial l-LTR and deletion containing r-LTR from RF3 and as occur in RF3X.

For the second procedure, the plasmids RFCΔS1-
B3L and RFAΔS1-B3L obtained above (containing the env
gene and part of the pol gene of FeLV-C and A, respectively) are digested with the restriction enzymes SstI
and XhoI and then isolated by gel electrophoresis. The
resultant larger fragments containing the cam$^R$ and amp$^R$
genes are then each ligated to an SstI/XhoI fragment
(containing a part of the FeLV-B pol gene) obtained by
similar SstI and XhoI digestion of pKC125 followed by
gel isolation. The resultant plasmids (p(C)-BΔ, p(A)-BΔ)
are then each digested with SstI, treated with alkaline
phosphatase and ligated to a gel isolated SstI fragment
derived from pKC125 and containing the gag gene and a
portion of the pol gene of FeLV-B. The plasmids
provided, designated pC-BΔ and pA-BΔ, each contain the
partial 1-LTR and deleted r-LTR of FeLV-B (from RF3 and
as occur in RF3X) and the intact FeLV-B gag and pol
genes, but the C and A env genes, and therefore immunogenicity, of FeLV-C and A, respectively.

Each hybrid proviral plasmid, containing the
modified LTRs of FeLV-B and either all the structural
genes (method 1) or the env gene only (method 2) of
FeLV-A or C, is then utilized to infect susceptible
tissue culture cells to produce modified hybrid FeLV
viruses and either modified, hybrid, proviral DNA or
virus used as a vaccine against FeLV-A or C infection.
Similar constructions are applicable to both possible,
additional FeLV subgroups and other pathological feline
viruses, e.g., feline rhinotracheitis virus, feline
infectious peritonitis virus etc.

The subject data show that the LTRs of retroviruses, including oncornaviruses, can be modified to
reduce replication capability, while retaining
infectivity, so as to provide effective vaccines for
mammalian or avian hosts subject to infection by such
viruses. Particularly, by modifying the enhancer region
in the LTR, preferably by a deletion of at least about 5 bases and modification of the other LTR to prevent complementarity, i.e., the reversion to wild-type activity by recombination, the infectivity can be retained and an immune response achieved by infecting (e.g., injection, inoculation) mammalian or avian hosts with either the modified viral DNA (proviral plasmid) or modified virus derived therefrom. In this manner, the host is exposed to the viral proteins in their normal environment to ensure that the immune response is activated toward the proper determinants of the wild-type virus. Furthermore, hybrid viral DNAs or viruses can be constructed containing the modified LTRs of one virus and at least the envelope gene(s) of another virus or other gene from a pathogen to provide an immune response, where reduced pathogenicity is dictated by the components of the first virus, but the immunogenicity is dictated by the exogenous gene. Such hybrid, modified viral DNA or viruses provide similar vaccines for mammalian or avian hosts susceptible to infection by the foreign pathogen.

Formation of the provirus can also provide for immunity from infection by the same or similar wild-type viruses, where the presence of an expressing provirus inhibits superinfection by the identical or similar virus. In addition, any change in cellular properties as a result of transcriptional regulation of a host gene by the transcriptional regulatory function of the LTR can be modified by diminishing or destroying the effect of the transcriptional regulation by the LTR sequence. The demonstrated attenuation of replication in tissue culture systems (Table 2) may be reflected in inoculated animals; thus, the inoculated animal may have a greater opportunity to develop an effective immune response.

Although the foregoing invention has been described in some detail by way of illustration and example for purposes of clarity of understanding, it will be obvious that certain changes and modifications may be practiced within the scope of the appended claims.

0156299

29

WHAT IS CLAIMED IS:

1.  A polynucleotide sequence capable of providing retroviral infection with diminished pathogenicity in a compatible host, said polynucleotide sequence comprising:

the structural genes necessary for replication of a retrovirus; and

a defective uncomplemented transcriptional regulatory system originating from in vitro modification, said defective system resulting in attenuated pathogenicity.

2.  A polynucleotide sequence according to any of Claims 1, 2 or 3, wherein said in vitro modification is a deletion of at least about five bases per strand.

3.  A polynucleotide sequence according to Claim 1 wherein said polynucleotide sequence is at least a part of FeLV which part includes the env gene of FeLV.

4.  A DNA polynucleotide sequence capable of providing retroviral infection with diminished pathogenicity, said polynucleotide sequence comprising:

the structural genes necessary for replication of a retrovirus;

a first LTR from the same or different retrovirus having at least a two base deletion per strand in the enhancer sufficient to inactivate the enhancer function region of said first LTR; and

any additional LTRs being modified so as to be incapable of restoration of said enhancer.

5.  An RNA polynucleotide sequence capable of providing retroviral infection with diminished pathogenicity in a compatible host, said RNA comprising:

the structural genes necessary for replication of a retrovirus,

the U3 and U5 regions modified by having at least one deletion in at least one of said regions of at least 5 nucleotides, wherein at least one of said deletions originates from in vitro modification, and results in attenuated pathogenicity.

6.    A polynucleotide sequence of about 475-500 bases per strand comprising the LTR of an FeLV.

7.    A polynucleotide sequence according to Claim 6, wherein said LTR is modified by a deletion of at least one base per strand in the region defining the enhancer.

8.    A vaccine comprising an effective amount of a polynucleotide sequence according to Claim 1, for immunizing a compatible host and a physiologically acceptable carrier.

9.    A vaccine according to Claim 8, wherein said polynucleotide sequence includes at least a part of an FeLV.

10.    A retrovirus preparation capable of providing infection with diminished pathogenicity in a compatible host, said virus preparation obtained by:

infecting a susceptible host with the polynucleotide sequence of Claim 1,

growing said host to produce retrovirus replication, and recovering said retrovirus from said host.

Nucleotide Homology Between Feline Leukemia Virus
Strain B and the MCF Strain of Murine Leukemia Virus
In the Region of the Long Terminal Repeat

Inverted Repeat
[B] AGAAAAA:GGGGGGAATGAAAGACCCCCTACCCCAAAATTTAGCCAGCTATTGC:AGTGGTGCCATTTCACA
    ******* ****************** *** * *** ** ***** *** ******* **
[M] AGAAAAAAGGGGGGGAATGAAAGACCCC::ACCTGTAGGTTTGGCAAGCTAGCTTAAGTAACGCCATTTTGCA

AGGCATGGAAAATTACTCAAGTATGTTCCCA::::T:GAGATACAAGG::::::::::::::::::::::::AA
************* *** ** * *    *    * **** *****                          **
AGGCATGGAAAAATACATAACTGAGAATAGAGAAGTTCAGAT:CAAGGTCAGGAACAGATGGAACAGCTGAA

        IEcoRVI
GTTAGAGGCTGAAACAGGATATCTGTGGTTAAGCA:::CCTGGGCCCCGGCTTGAGGCCAAGAACAGTTAAA
 ** *** ****************** ***** · *** ********* *********** *
::TATGGGC:CAAACAGGATATCTGTGGT:AAGCAGTTCCT::GCCCCGGCTCAGGGCCAAGAACAGATGGT

CCCCC:ATATAGCT:::::::::::::::::::::::::GAAACAGCAGAAGTTTCAAGGCCGCTGCCAGCAGTC
**** ** * *                          *** ** **** ***** *** *
CCCCAGATGC:GGTCCAGCCCTCAGCAGTTTCTAGAGAACCATCAGATGTTTCCAGGGTG::::::::::::

TCCAGGCTCCCCA:GTTGACCAGAGTTCGACCTTCCGCCTCATTTGAACTAACCAATCCCCACGCCTCTCGC
      ***** * **** ** * **** * **** *****************   *** ******
:::::::::CCCCAAG::GACCTGAAAT:GACCCTGTGCCTTATTTGAACTAACCAATCAGTTCGCTTCTCGC

                                                          >Cap Site
TTCTGTGCGCGCGCTTTCTGCT:::::::::::ATAAAACGAGCCCTCAGCCCCCAAC:GGGCGCGCAAGTC
****** ********* *****           ****** ****** ** ***   ** ******* ****
TTCTGTTCGCGCGCTT:CTGCTCCCCGAGCTCAATAAAA:GAGCCCACAACCCTTCACTCGGCGCGCCAGTC

TTTGCTGAGACTTGACCGCCCCGGGTACCCGTGTA:CGAATAAACC:TCTTGCTGTTTGCATCTGACTCGTG
 *      ****  ***** ********* *** * ******** ****** *** ***** **** ***
CTCCGATTGACTGAGTCGCCC:GGGTACCCGAGTATCCAATAAACCCTCTTGCAGTT:GCATCCGACTTGTG

                                                        Inverted Repea
GTCTCGGTGTTCCGTGGGTACGGGGTCTCATCGCCGAG:GAA:GACC:::::T:AGTTCGGGGGGTCTTTCA
****** ****** **** * ******* ** *** ** *** * ** ************
GTCTCGCTGTTCCTTGGG:A::GGGTCTCCTC::TGAGTGATTGACTACCCGTCAG::CGGGGGTCTTTCA

Fig. 1

Nucleotide Homology Between Feline Leukemia Virus

Strains A,B,C

in the Region of the Long Terminal Repeat

```
[C]  GAAGAAGGGGGGGAAATGAAAGACCCCCCCCCCCACCCCAAAACTTAGCCAGCTACTGCAGCAATGCCATTTCA
     *** ******** *    **    ****      ******** *****************  ** *******
[A]  GAAAAAGGGGGGGGATGAAAGACCCCCT:::::ACCCCAAAATTTAGCCAGCTACTGCAGTGGTGTCATTTCA
     ************ **************     ********************** ********* *******
[B]  GAAAAAGGGGGGGAATGAAAGACCCCCT:::::ACCCCAAAATTTAGCCAGCTATTGCAGTGGTGCCATTTCA

     CAAGGAATGGAAAATTACCCAAACATGTTCCCATGAGATATAAGGAAGTTAGGGGCTAAAACAGGATATCTG
     *****  ************ ***    *******************************  ***************
     CAAGGCATGGAAAATTACTCAAGTATGTTCCCATGAGATATAAGGAAGTTAGAGGCTAAAACAGGATATCTG
     ****************************************************  *****************  ***************
     CAAGGCATGGAAAATTACTCAAGTATGTTCCCATGAGATACAAGGAAGTTAGAGGCTGAAACAGGATATCTG

     TGGTTAAGCACCTGGGCCCCGGCTTAAAGCCAAGAACAGTTAAGCCTCGGATATAGCTGAAACAGCAGAAGT
     ***********************************  * **************** ** **************************
     TGGTTAAGCACCTGGGCCCCGGCTTGAGGCCAAGAACAGTTAAACCCCGGATATAGCTGAAACAGCAGAAGT
     ***********************************************  *********************
     TGGTTAAGCACCTGGGCCCCGGCTTGAGGCCAAGAACAGTTAAACCCCC:ATATAGCTGAAACAGCAGAAGT

     TTCAAGGCCACTGCCAGCAGTCTCCAGGCTCCCCAGTTGACCAGAGTTCAACCTTCCGCCTCATTTAAACTA
     ********* ************************************************  ********************
     TTCAAGGCCGCTGCCAGCAGTCTCCAGGCTCCCCAGTTGACCAGAGTTCGACCTTCCGCCTCATTTAAACTA
     *********************************************************** *****
     TTCAAGGCCGCTGCCAGCAGTCTCCAGGCTCCCCAGTTGACCAGAGTTCGACCTTCCGCCTCATTTGAACTA

     ACCAATCCCCACGCTTCTCGCTTCTGTACGCGCGCTTTCTGCTATAAAATGAGCCATCAGCCCCCACCGGGC
     ************** **********  *********************** ************** *****
     ACCAATCCCCACGCCTCTCGCTTCTGTGCGCGCGCTTTCTGCTATAAAACGAGCCATCAGCCCCCAACGGGC
     ******************************************************  ****************
     ACCAATCCCCACGCCTCTCGCTTCTGTGCGCGCGCTTTCTGCTATAAAACGAGCCCTCAGCCCCCAACGGGC

     GCGCAAGTCTTTGCTGAGACTTGACCGCCCCGGGTACCCGTGTACCGAATAAACCTCTTGCTGTTTGCATCT
     ************************************************ ***************** *******
     GCGCAAGTCTTTGCTGAGACTTGACCGCCCCGGGTACCCGTGTAGCGAATAAACCTCTTGCTGATTGCATCT
     **************************************************** *****************  *******
     GCGCAAGTCTTTGCTGAGACTTGACCGCCCCGGGTACCCGTGTA:CGAATAAACCTCTTGCTGTTTGCATCT

     GACTCGTGGTCTCGGTGTTCCGTGGGCACGGGGTCTCATCGCCGAGGAAGACCTAGTTCGGGGGTCTTTCA
     **** ***********************************************************************
     GACTTGTGGTCTCGGTGTTCCGTGGGCACGGGGTCTCATCGCCGAGGAAGACCTAGTTCGGGGGTCTTTCA
     **** ***************** ***************************************************
     GACTCGTGGTCTCGGTGTTCCGTGGGTACGGGGTCTCATCGCCGAGGAAGACCTAGTTCGGGGGTCTTTCA
```

Fig. 2

CONSTRUCTION OF ATTENUATED FeLV VIRUSES

FIG._3A.

FIG._3B.

FIG.—4.

FIG.—5.